# EUROPEAN PATENT SPECIFICATION

(11) **EP 0 554 446 B1**
(45) Date of publication and mention of the grant of the patent: **04.11.1998**
(21) Application number: 92919658.2
(22) Date of filing: 24.08.1992
(51) Int. Cl.: C12P 21/08, C07K 16/10, C12N 5/12, A61K 39/395

(54) **Monoclonal antibodies useful in therapy of HIV-1 infection**
In der Therapie von einer HIV-1-Infektion verwendbare monoklonale Antikörper
Anticorps monoclonaux utiles dans la thérapie d'une infection à HIV-1

(30) Priority: 22.08.1991 US 748562
(43) Date of publication of application: 11.08.1993
(73) Proprietor: NISSIN SHOKUHIN KABUSHIKI KAISHA, Osaka 532 (JP)
(72) Inventor: OHNO, Tsuneya, Boston, MA 02116 (US)
(74) Representative: Rackham, Anthony Charles
(86) International application number: US9207111
(87) International publication number: WO9304090

(56) References cited:
- EP-A- 0 295 803
- EP-A- 0 339 504
- EP-A- 0 465 979
- WO-A-88/09181
- WO-A-90/15078
- Aids Research and Human Retroviruses, Volume 6, No. 9, issued 1990, DURDA et al., "HIV-1 neutralizing monoclonal antibodies induced by a synthetic peptide", pages 1115-1123, see entire article.
- Science, Volume 250, issued 14 December 1990, JAVAHERIAN et al., "Broadly neutralizing antibodies elicited by the hypervariable neutralizing determinant of HIV-1", pages 1590-1594, see entire article.

## Description

The present invention relates, in general, to materials useful in the prevention and treatment of Human Immunodeficiency Virus (HIV-1) infection. More particularly, the invention relates to monoclonal antibodies useful in passive immunization of HIV-1 susceptible or infected animals, especially humans

The infective process of HIV-1 in vivo has recently been the subject of a review article by McCune, Cell, 64, pp. 351-363 (1991). Briefly, HIV-1 infects a variety of cell lineages, such as T-cells, monocytes/macrophages and neuronal cells, which express the CD4 receptor. Because the vast majority of CD4⁺ cells in the body are "resting" or quiescent and divide only in response to specific signals, infection with HIV-1 results in CD4⁺ cells harboring transcriptionally inactive virus. Stimulation of the immune system of infected animals, including active immunization, may result in polyclonal activation and the signaling of resting CD4⁺ cells to go into the S phase of the cell cycle. The replicating cells then actively produce viral particles, provoking spread of the infection. Considering this negative effect of stimulating the immune system of an HIV-1-infected animal, it is possible that the most effective method of preventing or treating HIV-1 infection is passive immunization, that is, administering anti-HIV-1 antibodies to a susceptible or infected animal.

Jackson et al., Lancet, 2, pp. 647-652 (1988) reports that a single administration of anti-HIV-I antibodies in the form of plasma to human patients afflicted with advanced acquired immunodeficiency syndrome (AIDS, the syndrome of progressive immune system deterioration associated with HIV-I infection) temporarily resulted in: fewer symptoms, a transient increase in T lymphocytes, a reduction in the frequency of opportunistic infections and a decline in the rate at which HIV-1 could be cultured from plasma or lymphocytes of the patients. See also, Karpas et al., Proc. Natl. Acad. Sci. USA, 85, pp. 9234-9237 (1988). Moreover, Emini et al., Nature, 355, pp. 728-730 (1992) reports that the administration of an antibody specifically reactive with HIV-1 to a chimpanzee before the animal was exposed to HIV-1 resulted in the chimpanzee remaining free of signs of viral infection. These studies indicate that antibodies capable of neutralizing HIV-1 can be useful in the prevention/treatment of HIV-1 infection.

The HIV-1 major external envelope glycoprotein, gp120, binds to the cellular CD4 receptor and facilitates the internalization of the virus. Several epitopes of the glycoprotein have been associated with the development of neutralizing antibodies. Ho et al., Science, 239, pp. 1021-1023 (1988) reports that amino acids 254-274 of gp120 elicit polyclonal antisera capable of group-specific neutralization of three different isolates of HIV-1. Conformation-dependent epitopes, epitopes not consisting of primary sequences of amino acids, on gp120 have also been implicated in eliciting antibodies that neutralize diverse strains of the virus by Haigwood et al., Vaccines 90, pp. 313-320 (1990) and Ho et al., J. Virol., 65(1), pp. 489-493 (1991). The so-called "principal neutralizing determinant" (PND) of HIV-1 gp120 has been localized to the "V₃ loop" of gp120. See Putney et al., Science, 234, pp. 1392-1395 (1986); Rusche et al., Proc. Natl. Acad. Sci. USA, 85, pp. 3198-3202 (1988); Goudsmit et al., Proc. Natl. Acad. Sci. USA, 85, pp. 4478-4482 (1988); Palker et al., Proc. Natl. Acad. Sci. USA, 85, pp. 1932-1936 (1988); and Holley et al., Proc. Natl. Acad. Sci. USA, 85 ,pp. 6800-6804 (1991). The V₃ loop consists of a hypervariable domain which is established by disulfide bonding between cysteine residues flanking the domain. The V₃ loop of HIV-1_{MN}, for example, is formed by a disulfide bond between the cysteine residues at positions 302 and 336 of gp120.

Recombinant and synthetic protein fragments including the series of amino acid residues of the V₃ loop from various HIV isolates have been reported to elicit isolate- or type-specific neutralizing antibodies in rodents by Lasky et al., Science, 233, pp. 209-212 (1986); Palker et al., supra; Matsushita et al., J. Virol., 62, pp. 2107-2114 (1988); and Javaherian et al., Proc. Natl. Acad. Sci. USA, 86, pp. 6768-6772 (1989). More recent studies [Putney et al., supra and LaRosa et al., Science, 249, pp. 932-935 (1990)] have demonstrated that the β-turn structure of the V₃ loop is the site recognized by the isolate-specific antibodies. Scott et al., Proc. Natl. Acad. Sci. USA, 87, pp. 8597-8601 (1990) report that the PND can also induce a type-specific antibody in humans. The hypervariability of the PND may account for the type-specific neutralizing activity generated by the epitope.

Several studies have suggested that antibodies prepared against recombinant gp120, purified gp120 or synthetic peptides from V₃ domain can neutralize diverse HIV-1 isolates. Javaherian et al., Science, 250, pp. 1590-1593 (1990) and Weiss et al., Nature, 324, pp. 572-575 (1986) each describe neutralization of both MN and III_{B} isolates by polyclonal sera from rabbits respectively immunized with a peptide corresponding to the PND of MN isolates and with a recombinant gp120 derived from a III_{B} isolate. See also, Haynes et al., U.S. Letters Patent 5,019,387.

Akerblom et al., AIDS, 4, pp. 953-960 (1990) describes monoclonal antibody preparations that neutralize III_{B} and eleven primary HIV-1 isolates. See also, PCT Published Patent Application No. WO 91/11198. The strain homology of the primary isolates is not determined, however, and the eleven isolates may also be III_{B}. Durda et al., AIDS Res. Hum. Retrov., 6, pp. 1115-1123 (1990) report a monoclonal antibody that blocks syncytia formation by both MN- and III_{B}-infected cells, but does not neutralize MN infectivity as determined by a "LAV capture immunoassay," an assay which is purported to give results that would correlate with reverse transcriptase activity. Patent Cooperation Treaty Patent Application No. WO 90/15078 of Scott et al., published on December 13, 1990, describes monoclonal antibodies which inhibit syncytium formation by cells infected with vaccinia virus expressing the PND of MN or "MN-like" isolates. None of the assertedly "broadly neutralizing" antibodies are demonstrated, by means of standard reverse transcriptase, p24 or MT-2 assays, to neutralize multiple strains of live HIV-1. See also, PCT Published Patent Application Nos. WO 88/09181 and WO 91/09625 and Liou et al., J. Immunol., 143(12), pp. 3967-3975 (1989).

The foregoing publications indicate that monoclonal antibodies reactive with the HIV-1 PND developed to date exhibit different levels of group reactivity, but may not have broad neutralizing activity. The different patterns of type- and group-specific reactivity indicated by these studies may be related to both the amino acid sequence and the conformation of the loop region of gp120.

Several studies have suggested that the CD4 receptor may not represent the only cellular receptor responsible for viral infectivity. The results of these studies raise the possibility that administering the heretofore described antibodies which block infection of CD4⁺ cells to a patient may afford only limited protection against HIV-1 infection. Cheng-Mayer et al., Proc. Natl. Acad. Sci. USA, 84, pp. 3526-3530 (1987) report HIV-1 infection of glial cells involving a receptor other than the CD4 molecule. Moreover, Takeda et al., Science, 242, pp. 580-583 (1988), indicate that antibody/HIV-1 complexes can infect monocytes by receptor-mediated endocytosis and enhance virus replication. Similar antibody-dependent enhancement of infection has been described in Halsted et al., Nature, 265, pp. 739-741 (1977); Peiris et al., Nature, 289, pp. 189-191 (1981); and Schlesinger et al., J. Immunol., 127, pp. 659-665 (1981).

Previous work has shown that certain animal viruses are inactivated by complement, particularly C1q, through an antibody-independent mechanism. See Weiss, in Molecular Biology of Tumor Viruses, RNA Tumor Viruses, Weiss et al., Eds., Cold Spring Harbor Laboratory, New York, pp. 1219-1220 (1982); Welsh et al., Virology, 74, pp. 432-440 (1976); Bartholomew et al., J. Exp. Med., 147, pp. 844-853 (1978); Cooper et al., J. Exp. Med., 144, pp. 970-984 (1976); and Sherwin et al., Int. J. Cancer, 21, pp. 6-11 (1978). While Banapour et al., Virology, 152, pp. 268-271 (1986) describe unheated serum preparations as having no effect on the density of HIV-1 or its ability to infect peripheral blood mononuclear cells, Spear et al., J. Virol., 64(12), pp. 5869-5873 (1990) report that HIV-1 treated with a combination of complement and pooled sera from HIV-1 sero-positive patients exhibits reduced infectivity.

There thus continues to exist a need in the art for new monoclonal antibody substances (including, e.g., murine-derived antibodies, humanized antibodies, and immunologically active antibody fragments) which are specifically immunoreactive with HIV-1. Ideally, such antibodies would be characterized by the ability to effect neutralization of multiple HIV-1 strains (e.g., III_{B} and MN) as determined by standard reverse transcriptase, p24, MT-2 and syncytium formation assays involving suitable cultured host cells (e.g., H9 cells). In view of projected use in passive immunization of infected and non-infected patients, such monoclonal antibodies would optimally be capable of participating in (i.e., mediating) complement-dependent virolysis of HIV-1 particles and antibody-dependent cytolysis of HIV-1 infected cells.

The present invention provides a monoclonal antibody characterized by the capacity of specifically binding to a sequence of amino acids of HIV-1 gp120 of gp160 protein consisting of the amino acid sequence G-P-G-R set out in SEQ ID NO:1 and the capacity of neutralizing, in vitro, the infection of H9 cells by live HIV-1 strains MN and III_{B} in reverse transcriptase, p24, MT-2 and syncytium formation assays and by comprising the six complementarity determining regions (CDRs) in the light and the heavy chains of monoclonal antibody NM01 which is produced by the hybridoma cell line having ATCC accession No. HB 10726

The products of the invention may be further characterized by their capacity to mediate complement-dependent virolysis of HIV-1 particles and/or antibody-dependent cellular cytotoxicity of HIV-1 infected cells.

Monoclonal antibodies according to the present invention, preferably IgG antibodies, are particularly suitable for use in anti-HIV-1 treatment of animals, especially humans, susceptible to or infected with HIV-1. Immunologically effective amounts of the monoclonal antibodies are administered to a patient infected with HIV-1 or at risk of infection with the virus to develop passive immunity to HIV-1 infection, and preferably, to effect complement-dependent virolysis of HIV-1 particles and/or antibody-dependent cellular cytotoxicity of HIV-1 infected cells in the patient.

Chimeric or "humanized" antibodies (including CDR-grafted antibodies), antibody fragments, and especially bi-specific antibodies based on the claimed monoclonal antibodies are within the contemplation of the present invention, as are recombinant antibody-related products produced in procaryotic or eucaryotic cells.

For example, antibody fragments, such as Fab and F(ab')₂ fragments, can be produced in culture by host cells such as E. coli, yeast, insect and mammalian cells upon determination of structural (sequence) information for the variable regions of the antibodies of the invention. Sequence information for the variable regions also enables preparation of CDR-grafted antibodies. Moreover, chimeric antibodies (e.g., mouse/human antibodies) may be prepared using transformed mouse myeloma cells or hybridoma cells and bi-specific antibodies may also be produced by hybrid hybridoma cells.

Also according to the invention is a pharmaceutical composition comprising an immunologically effective amount of a monoclonal antibody as described above and a pharmaceutically acceptable diluent, adjuvant or carrier, optionally together with other immunological agents and/or chemical therapeutic agents. Potential agents for combined administration include complement, antibodies which bind to various neutralizing and non-neutralizing domains of HIV-1 proteins, and chemical agents such as AZT.

As set forth in the following detailed description, monoclonal antibodies of the present invention were generated by immunization of an appropriate host with live HIV-1, thus presenting gp120 in its native conformation.

Specifically illustrating the present invention is the murine monoclonal antibody (designated NM-01) produced by hybridoma cell line HB 10726 which was received for deposit with the American Type Culture Collection, Rockville, Maryland, on April 9, 1991 and assigned A.T.C.C. Accession No. HB 10726.

Numerous aspects and advantages of the present invention will be apparent upon consideration of the illustrative examples and descriptions of practice of the present invention in the following detailed description thereof, reference being made to the accompanying drawings in which: FIGURE 1 is a composite autoradiogram of non-infected H9 cell, HIV-1_{MN} and HIV-1_{IIIB} proteins immunoblotted with a monoclonal antibody of the invention and immune sera from an sero-positive AIDS patient. FIGURE 2 graphically represents the results of immunoreactivity testing of an antibody of the invention with peptides corresponding to the V₃ loop region of different HIV-1 strains. FIGURES 3A to 3C, 4, 5, and 6A to 6B graphically report the results of the screening by reverse transcriptase, p24, MT-2 and syncytia formation assays, respectively, of a monoclonal antibody of the invention for the capacity to neutralize infection of H9 cells by live HIV-1 strains. FIGURE 7 graphically reports the results of an assay for determination of peptide blockage of neutralization of infectivity for an antibody of the invention. FIGURES 8A to B, 9A to F, and 10A to F are electron micrographs of HIV-1 particles which were treated with a combination of a monoclonal antibody of the invention and complement. FIGURES 11 and 12 set out the amino acid sequences of the variable regions of the light and heavy chains, respectively, of a monoclonal antibody of the present invention, NM-01, as well as the amino acid sequences of the light and heavy chains of a different anti-HIV-1 monoclonal antibody.

### EXAMPLES

The following examples illustrate practice of the invention in the production of a hybridoma cell line HB 10726, the isolation therefrom of monoclonal antibodies immuno-reactive with HIV-1 gp120 (or its precursor gp160) proteins as well as peptides comprising the amino acid sequence G-P-G-R set out in SEQ ID NO: 1, the characterization of such monoclonal antibodies.

More particularly, Example 1 is directed to the production of hybridoma cell line HB 10726 and the isolation of monoclonal antibody NM-01 therefrom. Example 2 relates to the mapping of the viral epitope recognized by antibody NM-01. Example 3 describes the characterization of the reactivity of the monoclonal antibody with diverse HIV-1 isolates. Example 4 relates to the screening of antibody NM-01 for the capacity to neutralize infection of H9 cells by various live HIV-1 strains as demonstrated by reverse transcriptase and p24 assays. Example 5 is directed to the further screening of the antibody for the capacity to neutralize infectivity of live HIV-1 isolates as demonstrated by MT-2 and syncytium formation assays. Example 6 relates to peptide blockage of HIV-1 infectivity neutralization properties of monoclonal antibody NM-01. Example 7 describes analysis of the capacity of monoclonal antibody NM-01 to mediate complement-dependent lysis of HIV-1. Example 8 relates to the determination of the effect of the combination of monoclonal antibody NM-01 and complement on HIV-1 infectivity of susceptible cells in culture. Example 9 describes the DNA and deduced amino acid sequences of the heavy and light chain variable regions of monoclonal antibody NM-01 and also relates to the preparation of a chimeric/humanized version of monoclonal antibody NM-01.

### Example 1

Hybridoma cell line HB 10726 was produced using standard immunological techniques such as described in Oi and Herzenberg, Selected Methods Cell Immunology, pp. 351-372 (1979) and Godding, J. Immunol. Meth., 39, pp. 285-308 (1980) and set out specifically below.

### Purification of live HIV-1_{MN}

300 ml of HIV-1_{MN}-infected H9 cell culture was collected and centrifuged at 1500 rpm for 5 minutes at 4°C to pellet the cells. The virus-containing supernatant was removed and saved, while the precipitate was recentrifuged at 2100 rpm for 20 minutes. The second supernatant was collected and pooled with the first, and the supernatant was ultracentrifuged in a SW 27 rotor at 25,000 rpm for 90 minutes at 4°C to pellet the viral particles. The resulting supernatant was discarded. The viral pellet was resuspended in approximately 10 ml TNE buffer (100 mM NaCl, 10 mM Tris-HCl, pH 7.7, 1 mM EDTA). An ultracentrifuge tube was prepared containing a bottom layer of 10 ml 50% sucrose TNE, a middle layer of 10 ml 25% sucrose TNE and a top layer of 10 ml virus sample, and was ultracentrifuged at 25,000 rpm at 4°C for 90 minutes. The virus precipitated as a white band between the layers of sucrose TNE and was collected with a pasteur pipet. 20 ml TNE/15 mM EDTA (100 mM NaCl, 10 mM Tris-HCl, pH 7.7, 15 mM EDTA) was added to the virus and the viral sample was spun again at 25,000 rpm at 4°C for 90 minutes. The resulting pellet comprised purified live HIV-1_{MN}.

### Immunization and hybridoma preparation

100 µg live HIV-1_{MN} was used to immunize each of three two-month old Balb/c mice by intraperitoneal injection. The mice were each boosted 3 weeks later with 30 µg virus and again after another 3 weeks with 100 µg of the viral preparation. The mice were sacrificed 3 days after the second boost and hybridoma cell lines were prepared by fusing splenocytes with P3-X63-Ag8-U1 cells (A.T.C.C. Accession Number CRL 1597). Hybridoma cells lines were also prepared from the spleens of mice immunized with chronically infected H9 cells (10 mice), acutely infected H9 cells (9 mice) and infected H9 cell membranes (3 mice). Chronically infected H9 cells are cells 2 to 3 weeks after infection having reverse transcriptase assay (RT) counts of 100,000 cpm to 150,000 cpm, while acutely infected H9 cells are cells 10 to 12 days after infection having RT counts of 200,000 cpm to 250,000 cpm.

The hybridoma cell lines were prepared by the following method. A mixture of spleen cells from immunized mice was spun at 800 g for 5 minutes. The supernatant was aspirated from the cell pellet and 1 ml warm (37°C) 50% PEG-1500 per 10⁸ cells was added to the pellet over a period of 1 minute (add 0.25 ml, stir gently with the pipet tip for 15 seconds and repeat). The mixture was stirred for an additional minute with the same pipet tip without breaking up cell clumps. 1 ml of "incomplete media" [RPMI 1640 (JRH Biosciences) supplemented with 25 mM HEPES (Sigma Co.), 10,000 U/ml penicillin and 10,000 mg/ml streptomycin] was then added over a period of 1 minute in the same manner (0.25 ml every 15 seconds) and another 1 ml was added over another minute. Next, 7 ml incomplete media was stirred in over a period of 2-3 minutes (1 ml every 20 seconds) resulting in a suspension of fine cell clumps. The final suspension was centrifuged at 500 g at setting 5 on a clinical centrifuge for 5 minutes and the supernatant was removed. The precipitate was resuspended by swirling (not vortexing or pipetting solution up and down) in "complete media" ["incomplete media" as above supplemented with 15% fetal calf serum (FBS)] to a concentration of 2x10⁶ cells per ml media. 0.1 ml of this suspension (2x10⁵ total cells) was plated per well of 96-well plates. The plates were incubated at 37°C, 7% CO₂. The day of fusion was considered Day 0.

### HAT selection and initial screening of hybridomas

24 hours after fusion (Day 1), 0.1 ml HAT media (10⁻⁴ M hypoxanthine, 5 X 10⁻⁷ M aminopterin and 1.6 X 10⁻⁵ M thymidine) was added to each well. On Days 2, 3, 5, 8, 11, 14, 17 and 21, 0.1 ml of media was removed from each well and replaced with fresh 0.1 ml HAT media. On Days 2 through 5, the wells appeared to contain only dead cells. Hybridomas began to appear between Days 5 and 10. The hybridomas were easily visible as colonies of very refractible cells surrounded by cellular debris.

### Hybridoma screening

Several assays were utilized for screening the hybridoma supernatants. Hybridomas secreting antibodies reactive with HIV-1 were initially identified by screening membranes prepared from non-infected and MN-infected H9 cells by ELISA with hybridoma culture supernatants. This initial screen was followed by immunofluorescence and radioimmunoassay screening to supplement the ELISA data with antibody binding data to live infected cells.

Cell membranes for the ELISA were prepared from infected or noninfected H9 cells. The cells were suspended in a 250 mM sucrose/10 mM Tris-HCl buffer at pH 7.4 containing 1 mM EDTA. The suspension was homogenized in a Dounce homogenizer placed in an ice bath until no viable cells were seen by Trypan Blue exclusion. The mixture was centrifuged for 2 minutes at 50 g. The resulting pellet was rehomogenized and recentrifuged. The two supernatants were combined and centrifuged at 20,000 g for 20 minutes. The pellet was again homogenized in the same buffer and centrifuged for 20 minutes and the pellet resuspended in 7 ml of the original 250 mM sucrose-EDTA buffer. This solution was then layered over a 2 M sucrose/10 mM Tris-HCl buffer containing 1 mM EDTA and centrifuged for 1 hour at 80,000 g. A fluffy white interface resulted which was collected and resuspended in the 250 mM sucrose buffer. Protein content was determined by the BCA assay (Pierce Chemical Company). The suspension was aliquoted and stored at -70°C.

For the ELISA, the cell membranes were added at a concentration of 400 ng/well to 96 well plates and were dried overnight at 25°C. The plates were washed with 0.5% Triton-X®/phosphate buffered saline (PBS), blocked with 5% fetal bovine serum (FBS)/PBS and washed again. Hybridoma supernatant (40 µl) was diluted in 50 µl PBS and added to the wells overnight at 4°C. After washing, rabbit anti-mouse IgG (H+L) conjugated to horseradish peroxidase (HRP) (Zymed) was added to the wells for 2 hours at 25°C. The wells were washed with 0.5% Triton-X®/PBS and then incubated in the presence of ABTS (Bio-Rad substrate kit) for 20 minutes before monitoring OD at 405 and 650 nm.

The supernatants of hybridomas generated from the spleen cells of mice immunized with chronically infected cells and acutely infected cells screened positive to both noninfected cell membrane and infected cell membrane in the ELISA, indicating that the antibodies produced by the hybridomas are not HIV-1-specific. Of 1039 hybridomas generated from the spleen cells of mice immunized with infected cell membranes, 5 of their supernatants reacted strongly with infected cell membrane and reacted very weakly with uninfected cell membrane. Western blots were performed on the supernatants from these hybridoma cells lines and it was determined that three of the monoclonal antibodies produced bound to HIV-1 p55, one bound to HIV-1 p55 and p24, and the last did not produce a band in the Western blot (data not shown).

1187 hybridomas were generated from the spleen cells of mice immunized with live HIV-1_{MN}. Four hybridoma cell lines were selected for further screening based on the results of an ELISA showing that antibodies in the four supernatants reacted strongly with infected cell membrane and very weakly with noninfected cell membrane. The results of the ELISA are presented in Table 1 as ratios of values obtained for infected cell membranes compared to uninfected cell membranes.

The supernatants of the four hybridomas were subjected to limited dilution cloning and were screened by radioimmunoassay (RIA). Rabbit anti-mouse IgG labelled with ¹²⁵I (R α M IgG-¹²⁵I) was purified on a Sephadex G-50 column (NEN-DuPont). Uninfected H9 cells or H9 cells (7.5x10⁵ cells in 150 µl) infected with HIV-1_{MN} were placed in 15 ml tubes. 50 µl supernatant from each hybridoma was added to each of the tubes containing the noninfected and infected cells then the mixtures were incubated overnight at 4°C. The cells were washed 2 times with 2 ml PBS/50% Tween-20® with vortexing between washes. 50 µl of the R α M IgG-¹²⁵I (750,000 cpm) in PBS/5% FBS was added and the mixture was again incubated overnight at 4°C. After incubation, the cells were washed 3 times with PBS/50% Tween-20®. 100 µl PBS/5% Triton-X® was added to disinfect the cells and 100 µl 1 M NaOH was added to help transfer the label to scintillation vials. The samples were counted and the results of the RIA are presented in Table 1 below as ratios between the cpm values obtained for infected cells compared to cpm values for uninfected cells.

**Table 1**

| ELISA | | | | |
|---|---|---|---|---|
| Ratio cpm inf/cpm uninf | | | | |
| Hybridoma Cell Line | Run 1 | Run 2 | Run 3 | RIA |
| 349 | 11.29 | 13.19 | 12.53 | 4.59 |
| 451 | 4.10 | 4.32 | 4.31 | 2.39 |
| 525 | 4.07 | 4.66 | 4.82 | 3.68 |
| HB 10726 | 5.76 | -- | -- | 9.81 |

Next, the four hybridoma cell lines were screened by immunofluorescence (IFA). 2 ml of either uninfected or HIV-1 infected H9 cells (approximately 1x10⁶ cells/ml) were placed a 10 ml sterile centrifuge tube with 10 ml PBS (without Ca⁺⁺ or Mg⁺⁺). The cells were washed once with 10 ml PBS by filling the tube, vortexing, spinning at 100 rpm for 5 minutes and aspirating all but about 100 µl supernatant leaving a "milky" cell suspension. While working in a laminar flow hood, 51 mm 10-well slides (Cell Line Association) were coated with cell suspension by flooding each well and then drawing the suspension back into the pipet tip. The coated slides were allowed to air dry and were then fixed in methanol at room temperature for 10 minutes. Supernatant from each of the four hybridomas was tested undiluted and at a 1:50 titer (supernatant diluted in 0.02% skim milk) for reactivity with slide preparations of uninfected and infected cells. 15 µl of undiluted or diluted supernatant was added to each slide well. The slides were incubated at 37°C for 30 minutes and submersed in PBS with stirring for 5 minutes. The slides were then quickly rinsed in distilled water and air dried in a laminar flow hood. 16 µl goat-anti-mouse IgG (H+L) F(ab)₂ fragment (Cappel Biomedical) diluted 1:80 in 0.02% skim milk was added to each well. The slides were again incubated at 37°C for 30 minutes and then submersed in PBS. The slides were rinsed in 0.01% Evans-blue solution in PBS for 5 seconds and rinsed 2 times in distilled water. The slides were examined by immunofluorescence and the results of the screening are presented in Table 2 wherein mouse IgG (MIgG), 5C5 antibody (anti-III_{B}), and Grp. 5 supernatant (from a hybridoma generated from the spleens of mice immunized with infected cell membranes; anti-infected and uninfected cells) are control antibodies.

**Table 2**

| IFA with live cells | | | |
|---|---|---|---|
| Antibodies | Uninf-H9 | III_{B}-H9 | MN-H9 |
| MIgG | - | - | - |
| 5C5 | - | + | - |
| Grp. 5 | +++ | +++ | +++ |
| 349 | - | - | +(+/-) |
| 451 | - | - | +/- |
| 525 | - | - | +(+/-) |
| HB 10726 | - | ++ | ++ |

The hybridoma cell line, HB 10726, was selected as the most promising antibody on the basis of the RIA and immunofluorescence data. The cell line did not have the highest binding ratio in the ELISA, but since the RIA and immunofluorescence results represent binding to live infected cells while the ELISA represents binding to dried cells membranes, the RIA data is more significant. The cell line was subcloned twice and the monoclonal antibody it produced was designated NM-01. Mice were intraperitoneally injected with the cell line by standard procedures and MAb NM-01 was concentrated from the ascites fluid by protein A affinity column purification (Pierce). The isotype of antibody NM-01 was determined to be IgG_{2b} by type specific antisera (Bio-Rad). The antibody (1.8 mg/ml) was diluted in RPMI 1640 medium with 15% FBS and utilized in the following examples.

### Example 2

In order to characterize the viral epitope recognized by monoclonal antibody NM-01, the antibody was first screened by Western blot analysis for reactivity with purified MN and III_{B} virion proteins and then by ELISA for reactivity with overlapping peptides corresponding to the amino acid sequence of the V3 loop region of HIV-1 gp120.

### Western blot analysis

MN and III_{B} virions purified from culture supernatants of infected H9 cells were disrupted in 1.3% SDS/3% β-mercaptoethanol and then subjected to electrophoresis in a 0.1% SDS/10% polyacrylamide gel. After transfer of the proteins to nitrocellulose paper, strips were incubated overnight with MAb NM-01 in blocking buffer (0.02 M Tris-HCl, pH 7.4, 0.1 M NaCl, 0.05% normal goat serum and 5% nonfat dry milk) at 4°C and then washed in 0.02 M Tris-HCl, pH 7.4, 0.1 M NaCl and 0.3% Tween®. The strips were then incubated with biotinylated goat anti-mouse IgG (Zymed) for 1 hour, washed and reacted with ¹²⁵I-Streptavidin (Amersham, Arlington Heights, IL) for an additional hour at 4°C. MAb NM-01 reactivity was monitored by autoradiography.

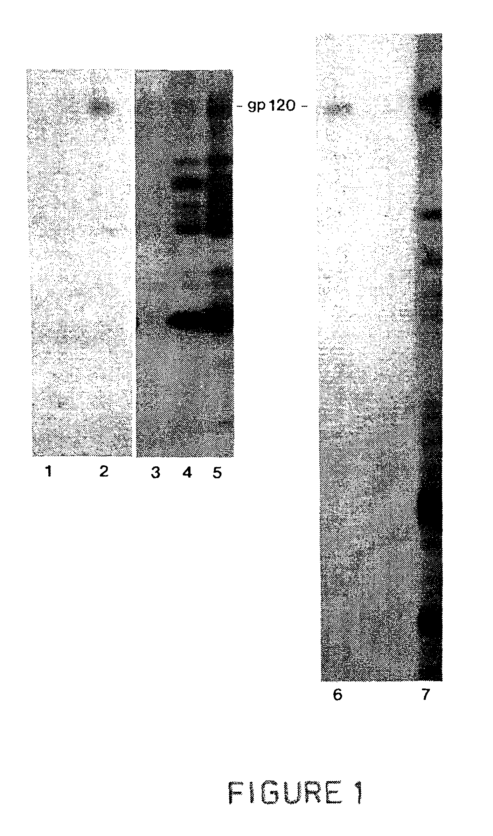

The autoradiographic results are presented in FIGURE 1 wherein: lanes 1 and 3 of the gel contained uninfected H9 cell membrane; lane 4 contained HIV-1_{MN} infected H9 cell membrane; lanes 2 and 5 contained HIV-1_{MN} virus; and lanes 6 and 7 contained HIV-1_{IIIB} virus. Antibody NM-01 was reacted with the proteins in lanes 1, 2 and 6 while HIV-1 sero-positive patient serum was reacted with the proteins in lanes 3-5 and 7.

Monoclonal antibody NM-01 exhibited reactivity with MN and III_{B} viral proteins having an apparent molecular weight of 120 kD, but did not react with any other viral antigens, indicating that the antibody recognizes an epitope of gp120.

### Epitope mapping by ELISA

To identify the specific epitope of gp120 recognized by antibody NM-01, the antibody was screened by ELISA for reactivity with overlapping peptides corresponding to the V₃ loop region of gp120. The peptides, synthesized by Multiple Peptide Systems, San Diego, CA, corresponded to amino acids 302-316, 312-326 and 322-336 of HIV-1_{MN} gp120.

The three peptides (250 ng/50 µl 0.1 M borate buffer, pH 8.0, per well) were incubated overnight at 37°C in Immulon 2 plates (Dynatech). The plates were washed with PBS and blocked with PBS/.1% Tween®/.1% Bovine Serum Albumin (BSA) for 1 hour at room temperature. The blocking agent was removed and differing amounts of antibody NM-01 or mouse IgG (MIgG), diluted in 100 µl HT media, were added to the plates. The antibody was allowed to react for 2 hours at room temperature. The plates were then washed 10 times with tap water. An HRP-conjugated rabbit anti-mouse second antibody, diluted 1:1000, was brought up in PBS/ .05% Tween®/.5% BSA, and 100 µl were added per well. The plates were incubated 1 hour at room temperature and then washed 10 times with tap water. ABTS substrate (Bio-Rad) was added for 20 minutes, and the plates were counted at 650 nm. SEQ ID NOs: 2-4 set out the amino acid sequences of the peptides and Table 3 sets out the results of the assay utilizing the overlapping peptides wherein the antibody MIgG and HT medium were negative controls.

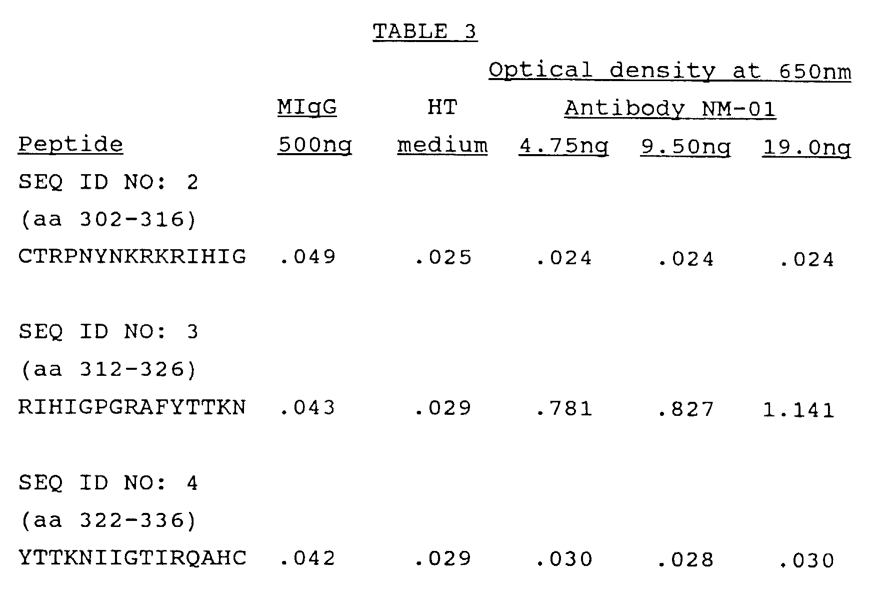

While there was no detectable reactivity over background of MAb NM-01 with the peptides corresponding to amino acids 302-316 or 322-336 of the V₃ loop, binding of the antibody to the peptide representing amino acids 312-326 was apparent. A control antibody, mouse IgG, did not bind to the peptides.

### Example 3

The demonstration that monoclonal antibody NM-01 binds to the V₃ loop region of HIV-1_{MN} gp120 prompted further studies on the extent of this reactivity with other HIV-1 isolates. The antibody was screened by ELISA for reactivity with peptides corresponding to the V₃ loop region of HIV-1 isolates III_{B}, RF, CDC4, NY/5, Z6, Z2 and ELI. The amino acid sequences of the peptides are set out below in Table 4 and in the sequence listing as SEQ ID NOs: 5-12, respectively.

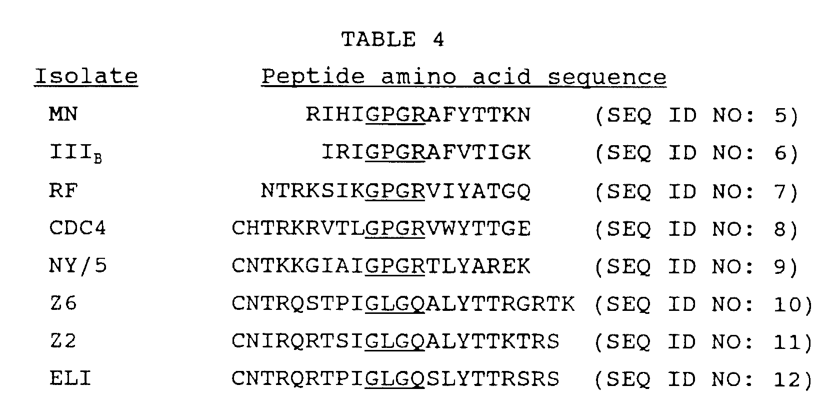

### Peptide binding assay

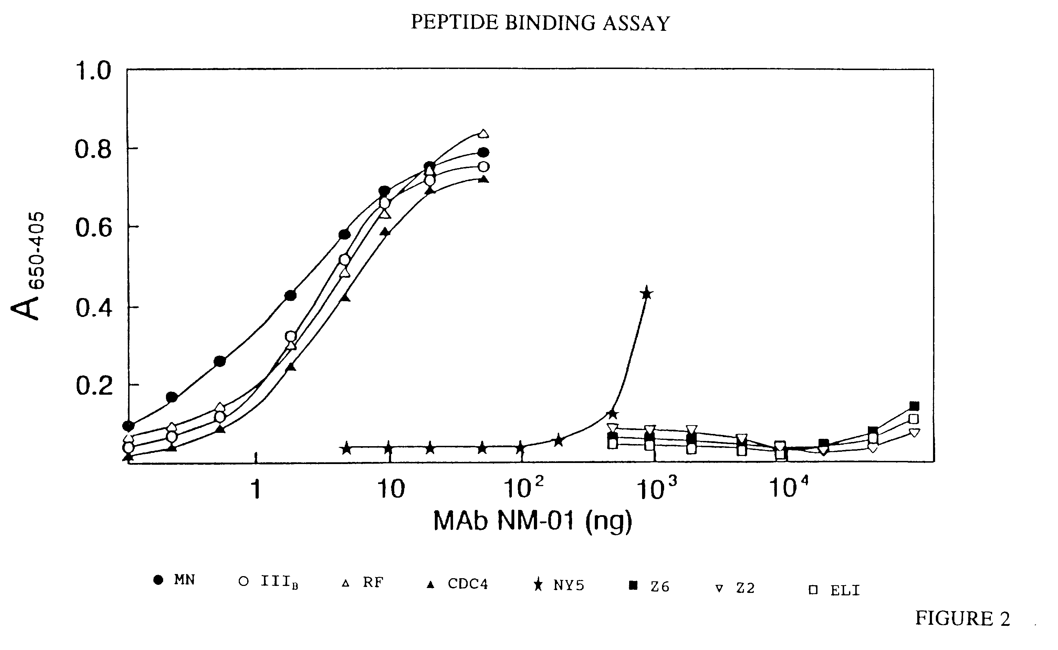

The peptides (250 ng/0.1 M borate buffer, pH 8.0, synthesized by American Biotechnologies, Cambridge, MA) were incubated overnight at 4°C in Immulon 2 plates (Dynatech). The plates were washed with PBS, blocked with 0.1% Tween®/0.1% BSA/PBS for 2 hours at 25°C and then incubated with MAb NM-01 for 1 hour at 37°C. After washing with tap water, the plates were incubated with HRP-conjugated rabbit anti-mouse second antibody for 1 hour at 25°C and then with ABTS substrate (Bio-Rad) for 20 minutes. Reactivity was determined by monitoring OD at 650-405 nm. The results of the assay are presented in FIGURE 2.

Monoclonal antibody NM-01 reacted with loop peptides from the MN (closed circle), III_{B} (open circle), RF (open triangle) and CDC4 (closed triangle) isolates. The binding of the antibody to the III_{B}, RF and CDC4 peptides was comparable to that obtained with the MN peptide. The antibody also showed a lesser affinity for the NY/5 peptide (star). Monoclonal antibody NM-01 is also putatively reactive with the RF-like peptide set out in SEQ ID NO: 13. In contrast, there was little, if any, reactivity with loop peptides from the Z6 (closed square), Z2 (inverted open triangle) and ELI (open square) isolates. These results indicate that monoclonal antibody NM-01 recognizes, in particular, an epitope of the V₃ loop of gp120 of multiple HIV-1 isolates having the amino acid sequence set in SEQ ID NO: 1, G-P-G-R.

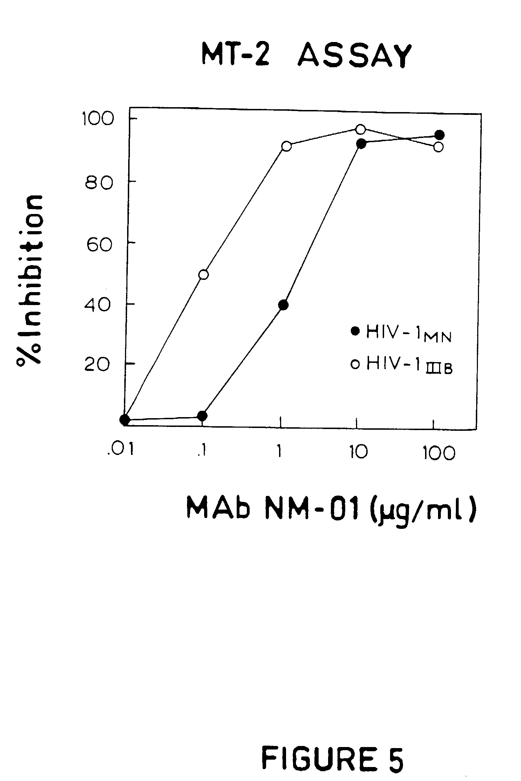

Another anti-HIV-1 gp120 monoclonal antibody, monoclonal antibody BAT123, is described in Liou et al., supra as being reactive with MN-like and III_{B}-like V₃ loop peptides and unreactive with an RF-like peptide (see Figure 5A on page 3972 of the article). These reported reactivities are different from those of monoclonal antibody NM-01 as described in the foregoing paragraph. While both antibodies NM-01 and BAT123 bind relatively well to III_{B} peptide, an approximately fifty-fold increase in the concentration of BAT123 is required to obtain binding to an MN peptide that is similar to the binding of NM-01. Moreover, NM-01 is reactive with the RF-like peptide set out in Table IV and SEQ ID NO: 7, while BAT123 does not bind to the RF-like peptide of SEQ ID NO: 13 even at antibody concentrations of 10,000 µg/ml.

### Example 4

Monoclonal antibody NM-01 was tested for the capacity to neutralize infection of H9 cells by live HIV-1 strains MN, III_{B}, and RF as measured by reverse transcriptase assay and HIV-1 strains MN and III_{B} as measured by p24 assay.

### Reverse transcriptase and p24 assays

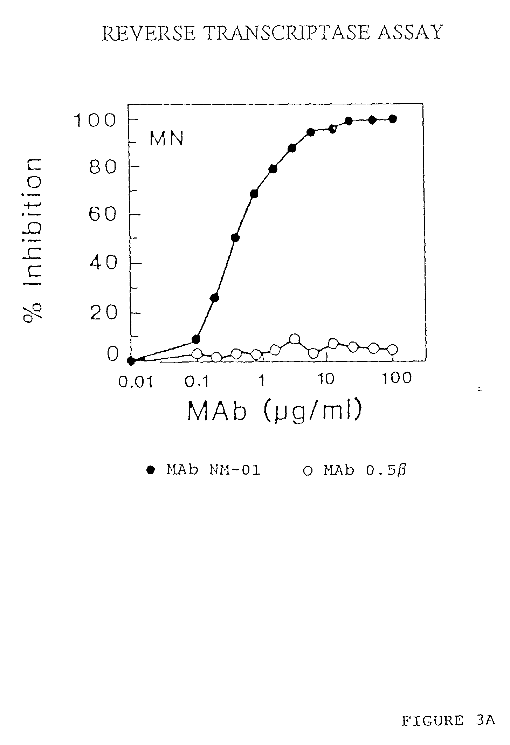

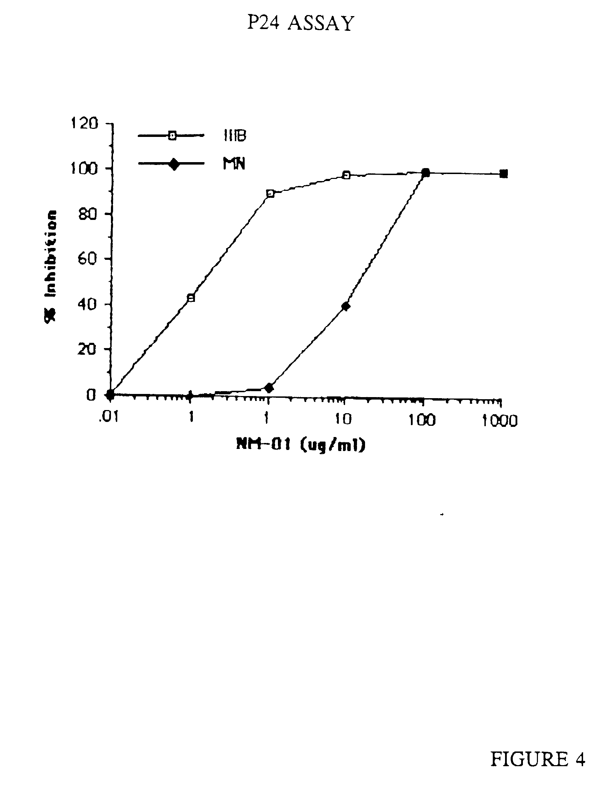

Dilutions of MAb NM-01 were incubated with 40 TCID₅₀ of MN or 100 TCID₅₀ of III_{B} live virus in 96-well plates for 1.5 hour at 37°C. MAb 0.5β (AIDS Research and Reference Reagent Program Catalog, National Institute of Allergy and Infectious Diseases) was used as both a positive and negative control in the RT studies; it binds to gp120 of HIV-1_{IIIB}. H9 cells (2.5 x 10⁴) were then added to each well and the plates were incubated for another hour at 37°C. The H9 cell suspension was then diluted in RPMI 1640/15% FBS and incubated in a 24 well plate at 37°C. Virus production was determined by reverse transcriptase (RT) assay performed on day 7 as described in Poiesz et al., Proc. Natl. Acad. Sci. USA, 77, pp. 7415-7419 (1980) and by p24 assay performed on day 5 (Dupont HIV-1 p24 Core Profile ELISA). Results of the two assays are presented in FIGURES 3A to 3B and 4, respectively.

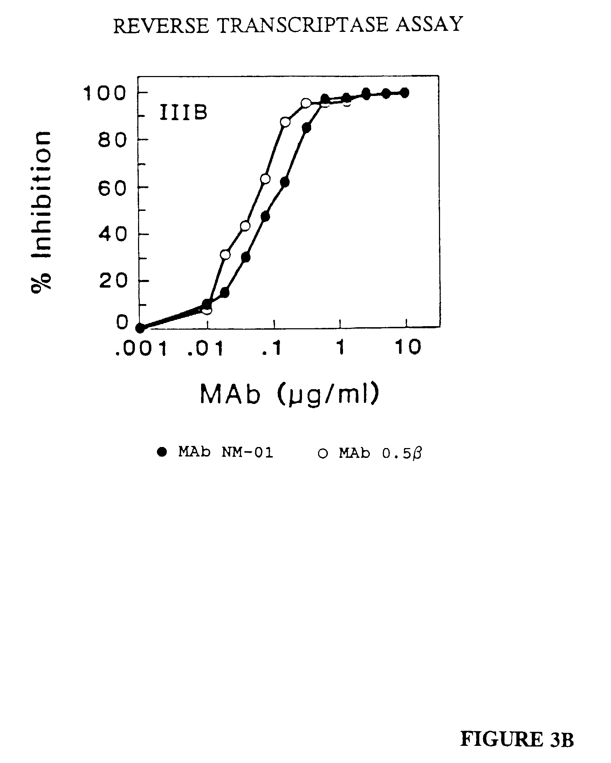

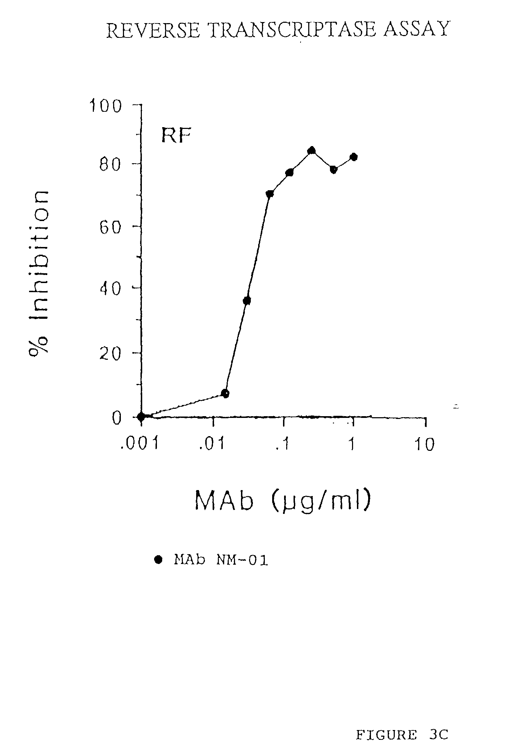

Monoclonal antibody NM-01 (closed circles in FIGURE 3A) completely neutralized infectivity of live MN virus, as determined by RT assay, at concentrations of 10-100 pµ/ml. Moreover, the use of the antibody at a concentration of <1 µg/ml resulted in 50% inhibition of viral infectivity (ID₅₀). These findings were in contrast to the absence of detectable neutralization with MAb 0.5β (open circles, FIGURE 3A). MAb NM-01 also neutralized live III_{B} virus at an ID₅₀ of approximately 0.1 µg/ml (FIGURE 3B). MAb 0.5β neutralized III_{B} slightly more effectively than MAb NM-01 (FIGURE 3B). Similar results were obtained for HIV-1 MN and III_{B} in the p24 assay. See FIGURE 4. In the reverse transcriptase assay, monoclonal antibody NM-01 also inhibited live RF virus at an ID₅₀ of about 0.05 µg/ml (FIGURE 3C).

These data indicate that the monoclonal antibody NM-01 neutralizes infectivity of at least three different strains of HIV-1.

### Example 5

Neutralization of live HIV-1 infectivity as demonstrated by reverse transcriptase and p24 assays was extended by studying the effects of monoclonal antibody NM-01 in MT-2 assays utilizing live MN and III_{B} virus and in syncytium formation assays utilizing live MN, III_{B} and RF virus.

### MT-2 assay

The MT-2 assay was performed as described in Richman, AIDS Research and Reference Reagent Program, Courier No. 90-01, pp. 6-9 (1990) with certain modifications. Live MN and III_{B} virus were incubated with dilutions of MAb NM-01 for 1.5 hours at 4°C in 96- well plates. MT-2 cells (8 x 10⁵) were added to the wells and the plates were incubated for 3 days at 37°C. MTT dye reduction according to Mosmann, J. Immunol. Meth., 65, pp. 55-63 (1983) and Pauwels et al., J. Virol. Meth., 20, pp. 55-63 (1983) was then performed to determine cell viability. Results of the MT-2 assay confirm the results of the reverse RT and p24 assays and are presented in FIGURE 5 wherein the open circles track values for III_{B} (100 TCID₅₀) and closed circles track values for MN (40 TCID₅₀).

Monoclonal antibody NM-01 neutralized the infectivity of live MN and III_{B} isolates at ID₅₀s of 2.0 and 0.1 µg/ml, respectively.

### Syncytium formation assay

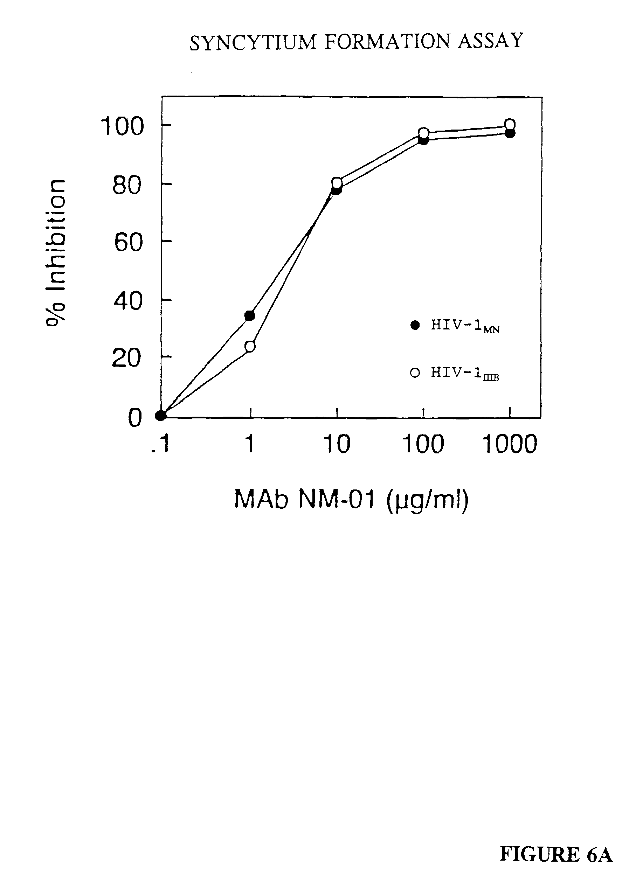

The binding inhibition assay was a modification of that described previously in Johnson and Walker, Eds., Techniques in HIV-1 Research, Stockton Press, New York, NY, pp. 92-97 (1990). Briefly, H9 cells chronically infected with either MN or III_{B} virus were incubated with dilutions of MAb NM-01 for 1 hour at 37°C. C8166 cells were then added to each well and incubated for 2 hours at 37°C. Syncytia greater than three lymphocyte cell diameters were counted and compared to that obtained for control infected H9 cells treated in the absence of antibody. Results of the syncytium formation assay also confirm the results of the RT and p24 assays and are presented in FIGURE 6A wherein open circles track values for III_{B} (100 TCID₅₀) and closed circles track values for MN (40 TCID₅₀). Monoclonal antibody NM-01 inhibited syncytium formation by MN-infected H9 cells at an ID₅₀ of 2 µg/ml, and by III_{B}-infected H9 cells at an ID₅₀ of 3 µg/ml.

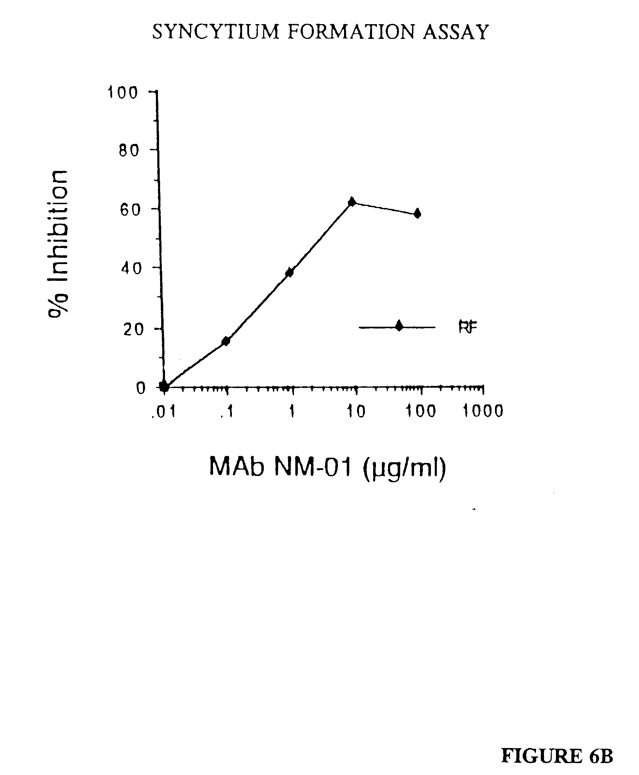

Corresponding syncytium formation inhibition results are presented for monoclonal antibody BAT123 in Table III of WO 88/09181. While 25 µg monoclonal antibody NM-01 inhibits about 85% of syncytium formation by MN-infected cells, 25 µg of BAT123 is reported to inhibit 51%, and while 25 µg NM-01 inhibits about 85% of syncytium formation by III_{B}-infected cells, BAT123 is reported to inhibit 77.8%. 25 µg BAT123 is also reported to inhibit syncytium formation by RF-infected cells by 51%. Monoclonal antibody NM-01 also inhibits syncytium formation by RF-infected cells (see FIGURE 6B). In the assay described in the foregoing paragraph, a concentration of 25 µg NM-01 inhibits about 59% of syncytium formation by RF-infected cells. Monoclonal antibody NM-01 inhibited syncytium formation by RF-infected cells at an ID₅₀ of 4 µg/ml.

Taken together, the results of the reverse transcriptase, p24, MT-2 and syncytia formation assays of Examples 4 and 5 indicate that monoclonal antibody NM-01 neutralizes binding and infectivity of diverse HIV-1 strains at concentrations less than 10 µg/ml.

### Example 6

In order to confirm that MAb NM-01 blocks infectivity of HIV-1 MN and III_{B} by binding to a portion of the gp120 V₃ loop, V₃ loop peptides were tested for the ability to block neutralization with the antibody.

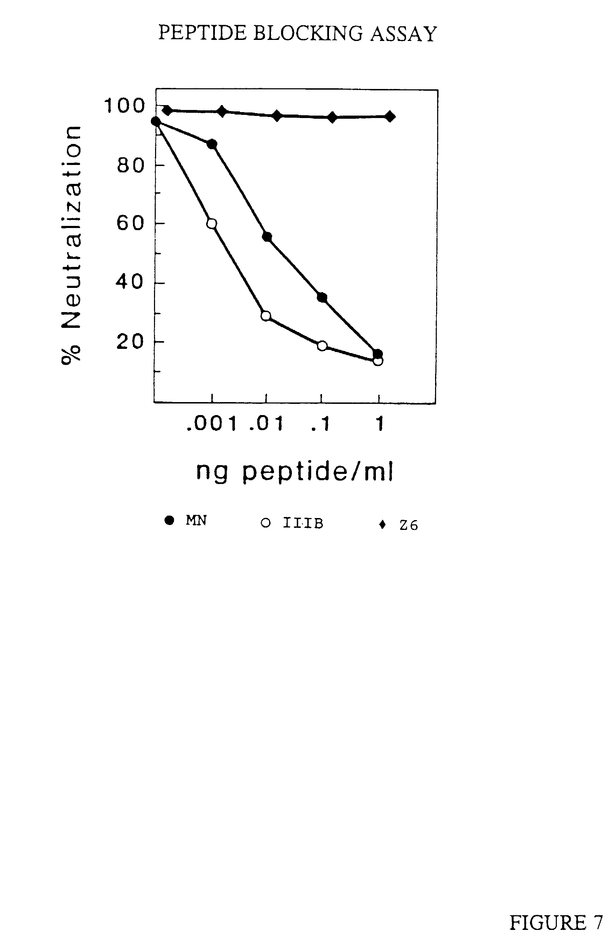

MAb NM-01 was incubated with the varying concentrations of peptides corresponding to the V₃ loops of the MN, III_{B} and Z6 strains (the sequences of the peptides are given in Table 4, above) for 30 minutes at 37°C before adding 100 TCID₅₀ of live III_{B} virus. H9 cells were then added for 1 hour and RT activity was determined after growth of the cells in complete medium for 7 days as described in Example 4. The results of the assay are presented in FIGURE 7.

While MAb NM-01 completely neutralized III_{B} infectivity at the lowest concentrations of peptide, this effect was progressively blocked by preincubation with increasing concentrations of MN (closed circle) and III_{B} (open circle) loop peptides. There was no detectable effect with similar concentrations of the peptide corresponding to the V₃ loop of the Z6 strain (closed diamond) which does not have the sequence of amino acids recognized by MAb NM-01. These results indicate that monoclonal antibody NM-01 blocks infectivity of HIV-1 by reacting with a specific portion of the gp120 V₃ domain.

### Example 7

Studies were also carried out to determine whether the monoclonal antibody NM-01 can activate the complement pathway and potentially destroy HIV-1 virions. Rabbit serum was used as a source of complement.

### Lysis of HIV-1 with MAb NM-01 and complement

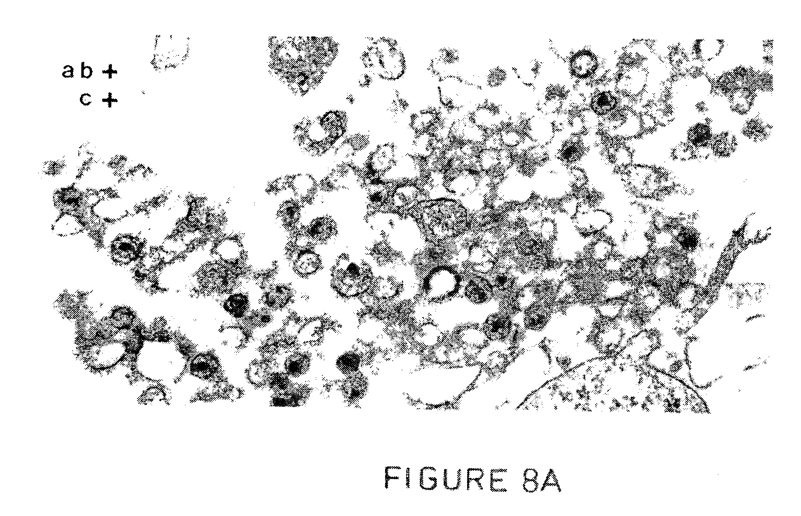

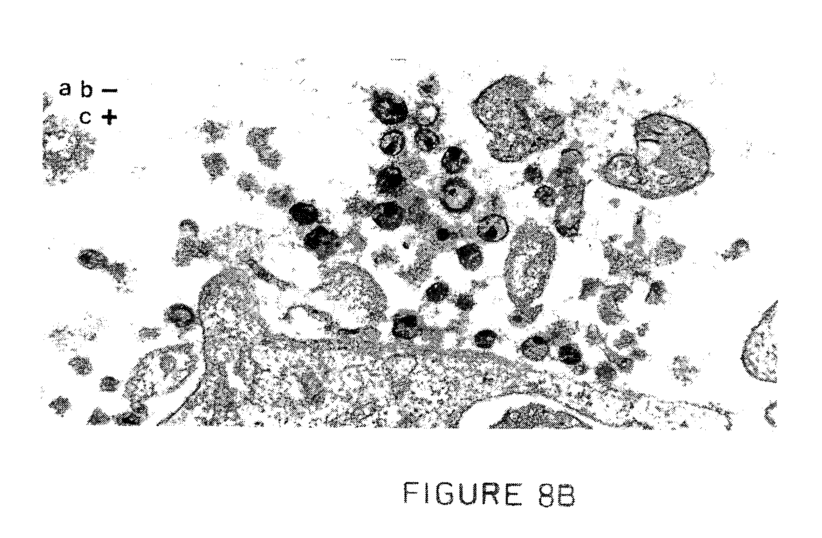

H9 cells infected with the HIV-1 III_{B} strain were washed in cytotoxicity medium (Cedarlane Lab. Ltd.). The cells were resuspended in cytotoxicity medium either in the absence or in the presence of 40 µg/ml MAb NM-01. After incubation for 2 hours at 4°C, rabbit complement (low-tox-MA; Cedarlane Lab. Ltd.) was added at a dilution of 1:6. The cell suspension was incubated at 4°C for 20 minutes and then 37°C for 45 minutes. The cells were doubly fixed with 2% glutaraldehyde/0.1 M phosphate buffer and 1% osmium tetroxide/0.1 M phosphate buffer. After embedding in epoxy resin, thin sections were cut and doubly stained with uranyl acetate and lead citrate. FIGURES 8, 9 and 10 are representative electron micrographs of the thin sections.

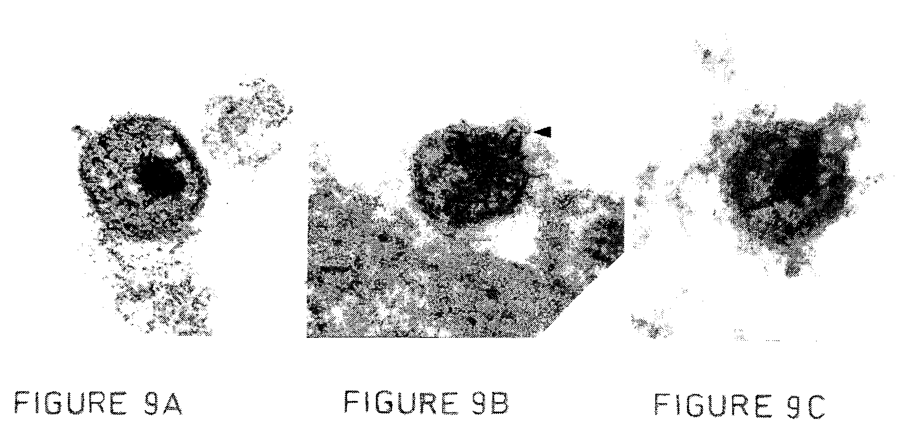

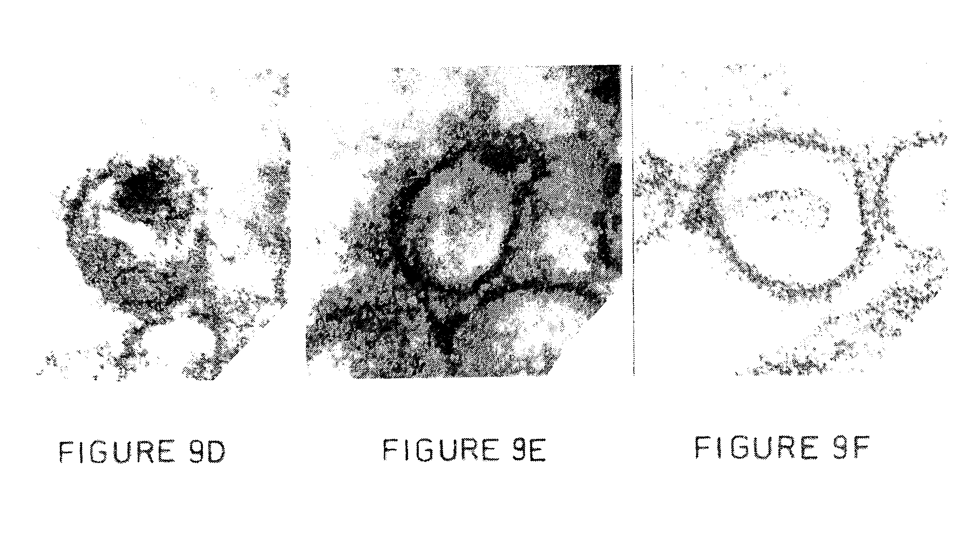

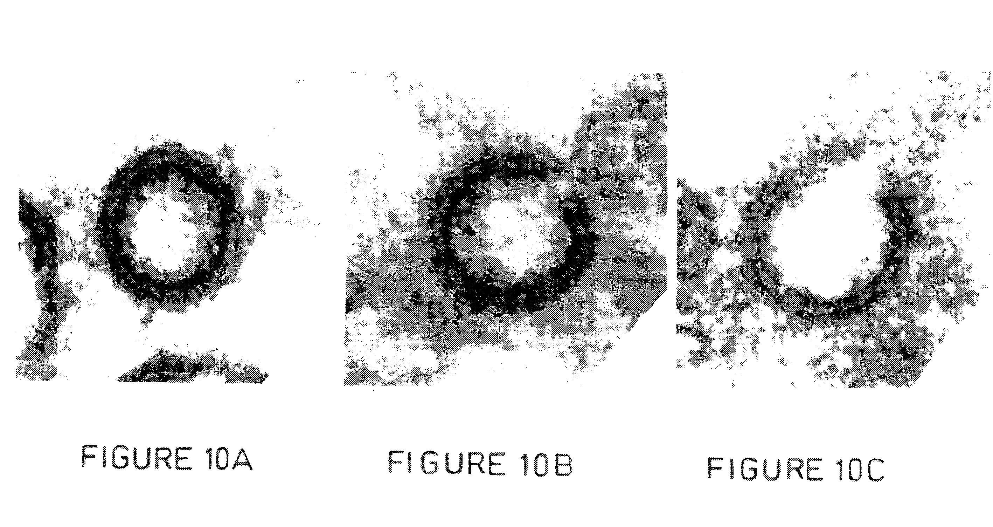

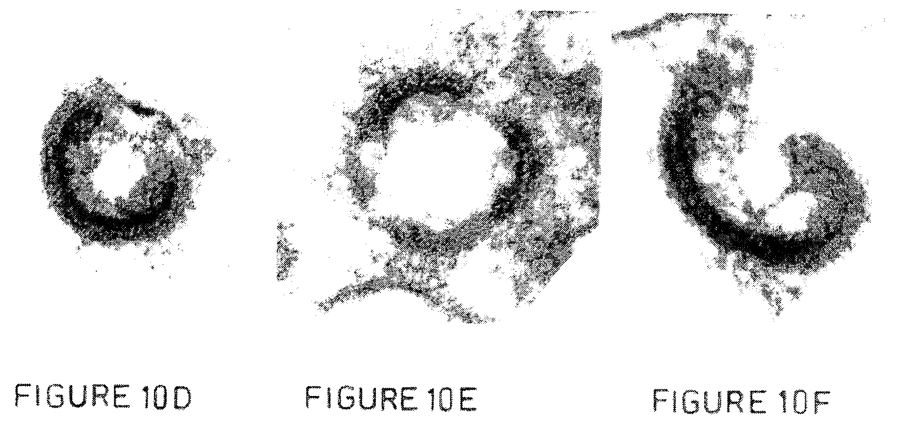

Rabbit serum alone (FIGURE 8B) and MAb NM-01 alone had no detectable effect on morphology of HIV-1. Exposure of HIV-1 to MAb NM-01 with complement was associated with the appearance of numerous viral particles with disrupted envelopes and loss of the electron dense core (FIGURE 8A). Representative preparations exhibited approximately 90% disrupted virions, the majority of which had loss of the internal core. The remaining 10% of virions were intact or had partially disrupted outer envelopes. Higher magnification revealed that disruption of HIV-1 occurred by direct lysis as illustrated in the series of micrographs of the lysis of mature and incomplete viral particles in FIGURES 9A to F and 10A to F, respectively.

### Example 8

The combination of monoclonal antibody NM-01 and complement was next analyzed to determine its effect on HIV-1 infectivity.

### Determination of tissue culture infectivity dose

HIV-1_{IIIB}-infected H9 cells were washed twice in cytotoxicity medium (Cedarlane Lab. Ltd.) and then resuspended in cytotoxicity medium containing 2 µg/ml MAb NM-01 or control IgG_{2b}. After incubation at 4°C for 2 hours, samples were aliquoted and either rabbit complement or heat-inactivated rabbit serum (Cedarlane Lab. Ltd.) was added at a dilution of 1:6. The cells were incubated at 4°C for 20 minutes and then 37°C for 45 minutes, washed with medium, resuspended in 50%FBS/RPMI 1640 medium and shaken. The supernatant or viral isolate was diluted 10-fold and then serially diluted 2 times before addition of 25 µl to H9 cells (1x10⁵/25 µl). After incubation for 3 hours at 37°C, the exposed cells were diluted with 10% FBS/RPMI 1640 medium and maintained at 37°C. Viral infection was determined after 6 days by reverse transcriptase assays. The tissue culture infectivity dose for 50% of H9 cell aliquots (TCID₅₀) was determined by the dilution that exhibited 50% infection. Table 5 sets out the results of the experiments.

**TABLE 5**

| MAb NM-01 | Complement | TCID₅₀ | | |
|---|---|---|---|---|
| | | Expt.1 | Expt.2 | Expt.3 |
| - | - | 1:2560 | 1:510 | 1:10240 |
| - | + | 1:5260 | 1:510 | 1:10240 |
| + | - | 1:320 | 1:128 | 1:1280 |
| + | + | 1:20 | 1:16 | 1:80 |

While monoclonal antibody NM-01 alone is capable of neutralizing infectivity of HIV_{IIIB}, treatment with both monoclonal antibody NM-01 and complement decreased infectivity of HIV_{IIIB} over 10-fold. These findings indicate that exposure of HIV-1 to both MAb NM-01 and complement is associated with a significant decrease in viral infectivity, and further support a role for MAb NM-01-mediated complement-dependent virolysis in HIV-1 therapy.

### Example 9

The variable regions of the heavy and light chain of monoclonal antibody NM-01 were cloned by PCR and sequenced. The DNA and deduced amino acid sequences of the NM-01 heavy and light chain variable regions are set out in SEQ ID NOs: 14 and 15 and SEQ ID NOs: 16 and 17, respectively. Nucleotides 1-21 and 334-363 of SEQ ID NO: 16 correspond to the PCR primers used to amplify NM-01 light chain sequences and nucleotides 1-27 and 385-402 of SEQ ID NO: 14 correspond to the PCR primers used to amplify NM-01 heavy chain sequences.

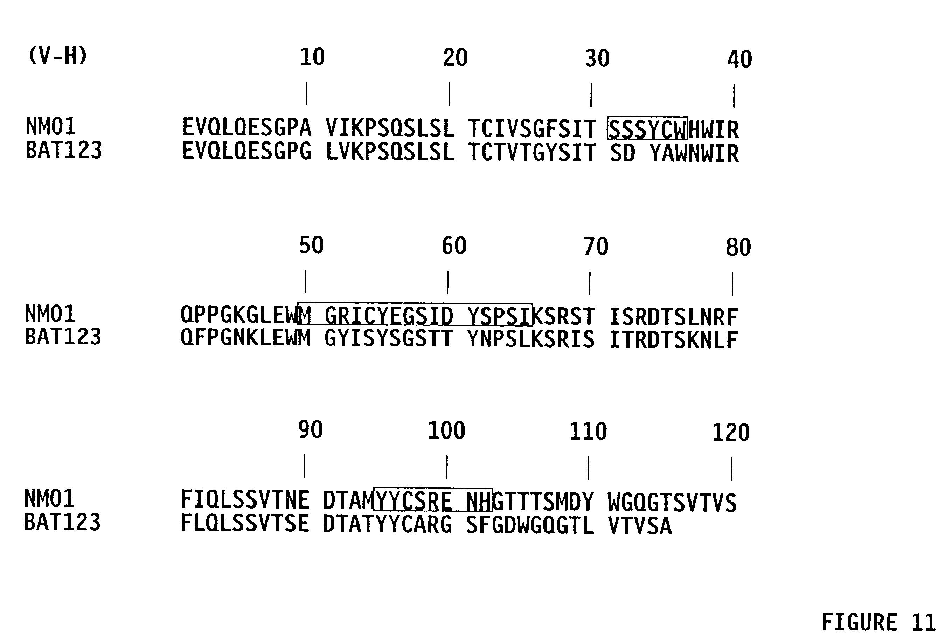

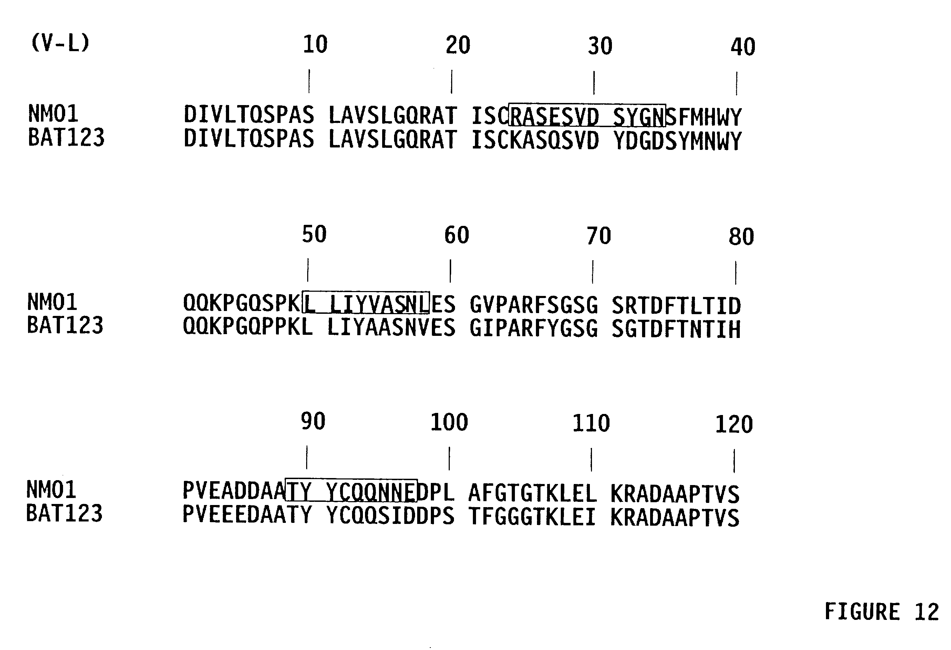

The first 120 residues of the amino acid sequences of the NM-01 heavy and light chain variable regions are also set out in FIGURES 11 and 12, respectively, wherein the boxed amino acids are the complementarity determining regions (CDRs) of the antibody which determine the binding specificity of the antibody. In the FIGURES, each heavy or light chain amino acid sequence is compared to the corresponding amino acid sequence of the variable regions of monoclonal antibody BAT123 as reported in Liou et al., supra. The heavy chain variable region of NM-01 differs from that of BAT-123 by forty-four amino acids out of a total of one hundred twenty. The light chain variable regions of the two antibodies differ by twenty-four amino acids. Significantly, the three CDRs in the heavy chain (V-H) of the NM-01 molecule are 44 to 50% different in sequence from those of BAT-123, while the sequences of the three CDRs in the light chain (V-L) vary by 22%-45%. Therefore, it is clear from analysis of the structures of NM-01 and BAT123 that the two are different antibodies.

Based on the DNA sequence information presented in SEQ ID NOs: 14 and 16, a humanized version of the NM-01 antibody may be prepared according to the methods of Tempest et al., BIO/TECHNOLOGY, 9, 266-271, (1991) and Riechmann et al., Nature, 322, 323-327 (1988). Briefly, DNA sequences encoding the six CDRs of the light and heavy chains of monclonal antibody NM-01 may be introduced into DNA constructs encoding appropriate human heavy or light chain framework regions. The resulting constructs encoding complete heavy and light chain variable domains are then co-expressed in a cell with DNA sequences encoding human constant domains to produce a humanized antibody with the binding specificity of monoclonal antibody NM-01.

## Claims

1. A monoclonal antibody characterized by the capacity of specifically binding to a sequence of amino acids of HIV-1 gp120 of gp160 protein consisting of the amino acid sequence G-P-G-R set out in SEQ ID NO:1 and the capacity of neutralizing, in vitro, the infection of H9 cells by live HIV-1 strains MN and III_{B} in reverse transcriptase, p24, MT-2 and syncytium formation assays and by comprising the six complementarity determining regions (cDRs) in the light and the heavy chains of monoclonal antibody NM01 which is produced by the hybridoma cell line having ATCC accession No. HB 10726.

2. A monoclonal antibody as claimed in Claim 1 further characterized by the capacity of mediating complement-dependent virolysis of HIV-1 particles.

3. A monoclonal antibody as claimed in Claim 1 further characterized by the capacity of mediating antibody-dependent cellular cytotoxicity of HIV-1 infected cells.

4. A monoclonal antibody as claimed in any preceding claim further characterized by being a chimeric antibody.

5. A monoclonal antibody as claimed in any of claims 1 to 3 further characterized by being a murine antibody.

6. A monoclonal antibody as claimed in any of claims 1 to 3 further characterized by being an IgG antibody.

7. A hybridoma cell line which secretes a monoclonal antibody as claimed in any preceding claim.

8. The hybridoma cell line having ATCC Accession No. HB 10726.

9. The monoclonal antibody, NM-01, produced by the hybridoma cell line as claimed in Claim 8.

10. A pharmaceutical composition comprising an immunologically effective amount of a monoclonal antibody as claimed in any of claims 1 to 6 and 9, and a pharmaceutically acceptable diluent, adjuvant or carrier and/or other immunological agent and/or chemical therapeutic agent.

11. A pharmaceutical composition as claimed in Claim 10 further comprising an immunologically effective amount of complement.

12. A pharmaceutical composition as claimed in Claim 11 in that the complement is provided as serum.

13. The use of a monoclonal antibody as claimed in any of claims 1 to 6 and 9 for the manufacture of a medicament for the treatment of animals susceptible to or infected with HIV-1.

14. The use of a pharmaceutical composition as claimed in any of claims 10 to 12 for the manufacture of a medicament for therapeutic application to HIV-1 infection.

15. An antibody fragment comprising the variable region of a monoclonal antibody as claimed in any of claims 1 to 6 and 9.

16. An antibody fragment as claimed in Claim 15 further characterized by being a fragment of a murine monoclonal antibody.

## Patentansprüche

1. Ein monoklonaler Antikörper, gekennzeichnet durch die Fähigkeit zur Bindung speziell an eine Sequenz von Aminosäuren des gp120-Proteins von HIV-1 des gp160-Proteins, das die Aminosäure-Sequenz G-P-G-R enthält, die in SEQ ID No:1 dargestellt ist, und durch die Fähigkeit zur In-vitro-Neutralisierung der Infektion von H9-Zellen mit lebenden HIV-1-Stämmen MN und III_{B} in Umkehrtranscriptase-, p24-, MT-2- und Syncytiumbildungsassays sowie dadurch gekennzeichnet, daß er die sechs Kemplementaritäts-bestimmenden Regionen (CDRs) im Licht und die schweren Ketten des monoklonalen Antikörpers NM01 enthält, der durch die Hybridom-Zellinie erzeugt wird, die die ATCC Accession No. HB 10726 aufweist.

2. Ein monoklonaler Antikörper nach Anspruch 1, ferner gekennzeichnet durch die Fähigkeit, durch Vermittlung eine Komplement-abhängige Virolyse von HIV-1-Partikeln herbeizuführen.

3. Ein monoklonaler Antikörper nach Anspruch 1, ferner gekennzeichnet durch die Fähigkeit, durch Vermittlung eine Antikörper-abhängige zellulare Zytotoxizität von mit HIV-1 infizierten Zellen herbeizuführen.

4. Ein monoklonaler Antikörper nach einem der vorstehenden Ansprüche, ferner dadurch gekennzeichnet, daß er ein chimärer Antikörper ist.

5. Ein monoklonaler Antikörper nach einem der Ansprüche 1 bis 3, ferner dadurch gekennzeichnet, daß er ein muriner Antikörper ist.

6. Ein monoklonaler Antikörper nach einem der Ansprüche 1 bis 3, ferner dadurch gekennzeichnet, daß er ein IgG-Antikörper ist.

7. Eine Hybridom-Zellinie, die entsprechend einem der vorstehenden Ansprüche einen monoklonalen Antikörper erzeugt.

8. Die Hybridom-Zellinie, die die ATCC Accession No. HB 10726 aufweist.

9. Der monoklonale Antikörper NM-01, der entsprechend Anspruch 8 durch die Hybridom-Zellinie erzeugt wird.

10. Eine pharmazeutische Zusammensetzung, die entsprechend einem der vorstehenden Ansprüche 1 bis 6 und 9 eine immunologisch wirksame Menge eines monoklonalen Antikörpers und ein pharmazeutisch geeignetes Verdünnungsmittel, Adjuvans oder eine solche Trägersubstanz und/oder einen anderen immunologisch wirksamen Stoff und/oder ein chemisches Therapeutikum enthält.

11. Eine pharmazeutische Zusammensetzung nach Anspruch 10, die des weiteren eine immunologisch wirksame Menge eines Komplements enthält.

12. Eine pharmazeutische Zusammensetzung nach Anspruch 11, dadurch gekennzeichnet, daß das Komplement als Serum zur Verfügung steht.

13. Der Einsatz eines monoklonalen Antikörpers nach einem der Ansprüche 1 bis 6 und 9 zur Herstellung eines Medikaments zur Behandlung von Tieren, bei denen das Risiko einer HIV-1-Infektion besteht oder die mit HIV-1 infiziert sind.

14. Der Einsatz einer pharmazeutischen Zusammensetzung nach einem der Ansprüche 10 bis 12 zur Herstellung eines Medikaments zur therapeutischen Anwendung bei einer HIV-1-Infektion.

15. Ein Antikörperfragment, das entsprechend den Ansprüchen 1 bis 6 und 9 die variable Region eines monoklonalen Antikörpers enthält.

16. Ein Antikörperfragment nach Anspruch 15, das des weiteren dadurch gekennzeichnet ist, daß es ein Fragment eines murinen monoklonalen Antikörpers ist.

## Revendications

1. Anticorps monoclonal, caractérisé par la capacité de se lier spécifiquement à une séquence d'aminoacides gp120 de HIV-1 de la protéine GP160, constituée de la séquence d'aminoacides G-P-G-R présentée dans SEQ ID NO:1 et la capacité de neutraliser, in vitro, l'infection des cellules H9 par les souches de HIV-1 vivantes MN et IIIB dans des essais par transcriptase inverse, p24, MT-2 et de formation de syncytium, et par le fait de comprendre les six régions déterminant la complémentarité (CDR) dans les chaînes légères et lourdes de l'anticorps monoclonal NM01 produit par la lignée de cellules hybridomes ayant le numéro d'accès ATCC HB 10726.

2. Anticorps monoclonal selon la revendication 1, caractérisé en outre par la capacité d'assurer la médiation de la virolyse dépendant du complément des particules de HIV-1.

3. Anticorps monoclonal selon la revendication 1, caractérisé en outre par la capacité d'assurer la médiation de la cytotoxicité cellulaire dépendant d'anticorps des cellules infectées par HIV-1.

4. Anticorps monoclonal selon l'une quelconque des revendications précédentes, caractérisé en outre par le fait d'être un anticorps chimérique.

5. Anticorps monoclonal selon l'une quelconque des revendications 1 à 3, caractérisé en outre par le fait d'être un anticorps murin.

6. Anticorps monoclonal selon l'une quelconque des revendications 1 à 3, caractérisé en outre par le fait d'être un anticorps IgG.

7. Lignée de cellules hybridomes, sécrétant un anticorps monoclonal tel que revendiqué selon l'une quelconque des revendications précédentes.

8. Lignée de cellules hybridomes ayant le numéro d'accès ATCC HB 10726.

9. Anticorps monoclonal, NM01, produit par la lignée de cellules hybridomes selon la revendication 8.

10. Composition pharmaceutique comprenant une quantité immunologiquement efficace d'un anticorps monoclonal selon l'une quelconque des revendications 1 à 6 et 9, et un diluant, adjuvant ou support pharmaceutiquement acceptable et/ou un autre agent immunologique et/ou un agent thérapeutique chimique.

11. Composition pharmaceutique selon la revendication 10, comprenant en outre une quantité immunologiquement efficace de complément.

12. Composition pharmaceutique selon la revendication 11, caractérisée en ce que le complément est fourni sous forme de sérum.

13. Utilisation d'un anticorps monoclonal selon l'une quelconque des revendications 1 à 6 et 9, pour la fabrication d'un médicament pour le traitement d'animaux sensibles à ou infectés par HIV-1.

14. Utilisation d'une composition pharmaceutique selon l'une quelconque des revendications 10 à 12 pour la fabrication d'un médicament pour l'application thérapeutique à une infection par HIV-1.

15. Fragment d'anticorps comprenant la région variable d'un anticorps monoclonal selon l'une quelconque des revendications 1 à 6 et 9.

16. Fragment d'anticorps selon la revendication 15, caractérisé en outre par le fait d'être un fragment d'un anticorps monoclonal murin.


### SEQUENCE LISTING

### (1) GENERAL INFORMATION:

(i) APPLICANT: Ohno, Tsuneya
(ii) TITLE OF INVENTION: HIV Immunotherapeutics
(iii) NUMBER OF SEQUENCES: 17
(iv) CORRESPONDENCE ADDRESS:
   (A) ADDRESSEE: Marshall, O'Toole, Gerstein, Murray & Bicknell
   (B) STREET: Two First National Plaza, 20 South Clark Street
   (C) CITY: Chicago
   (D) STATE: Illinois
   (E) COUNTRY: USA
   (F) ZIP: 60603
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE:
   (C) CLASSIFICATION:
(vii) PRIOR APPLICATION DATA:
   (A) APPLICATION NUMBER: US 07/748,562
   (B) FILING DATE: 22-AUG-1991
(viii) ATTORNEY/AGENT INFORMATION:
   (A) NAME: Noland, Greta E.
   (B) REGISTRATION NUMBER: 35,302
   (C) REFERENCE/DOCKET NUMBER: 31016
(ix) TELECOMMUNICATION INFORMATION:
   (A) TELEPHONE: (312) 346-5750
   (B) TELEFAX: (312) 984-9740
   (C) TELEX: 25-3856

### (2) INFORMATION FOR SEQ ID NO:1:

(i) SEQUENCE CHARACTERISTICS:
   (A) LENGTH: 4 amino acids
   (B) TYPE: amino acid
   (D) TOPOLOGY: linear
(ii) MOLECULE TYPE: peptide
(xi) SEQUENCE DESCRIPTION: SEQ ID NO:1:
   Gly Pro Gly Arg
   1

### (2) INFORMATION FOR SEQ ID NO:2:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(xi) SEQUENCE DESCRIPTION: SEQ ID NO:2:
   Cys Thr Arg Pro Asn Trp Asn Lys Arg Lys Arg Ile His Ile Gly
   1               5                   10                  15

### (2) INFORMATION FOR SEQ ID NO:3:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(xi) SEQUENCE DESCRIPTION: SEQ ID NO:3:
   Arg Ile His Ile Gly Pro Gly Arg Ala Phe Tyr Thr Thr Lys Asn
   1               5                   10                  15

### (2) INFORMATION FOR SEQ ID NO:4:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(xi) SEQUENCE DESCRIPTION: SEQ ID NO:4:
   Tyr Thr Thr Lys Asn Ile Ile Gly Thr Ile Arg Gln Ala His Cys
   1               5                   10                  15

### (2) INFORMATION FOR SEQ ID NO:5:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(xi) SEQUENCE DESCRIPTION: SEQ ID NO:5:
   Arg Ile His Ile Gly Pro Gly Arg Ala Phe Tyr Thr Thr Lys Asn
   1               5                   10                  15

### (2) INFORMATION FOR SEQ ID NO:6:

(i) SEQUENCE CHARACTERISTICS:
   (A) LENGTH: 14 amino acids
   (B) TYPE: amino acid
   (D) TOPOLOGY: linear
(ii) MOLECULE TYPE: peptide
(xi) SEQUENCE DESCRIPTION: SEQ ID NO:6:
   Ile Arg Ile Gly Pro Gly Arg Ala Phe Val Thr Ile Gly Lys
   1               5                   10

### (2) INFORMATION FOR SEQ ID NO:7:

(i) SEQUENCE CHARACTERISTICS:
   (A) LENGTH: 19 amino acids
   (B) TYPE: amino acid
   (D) TOPOLOGY: linear
(ii) MOLECULE TYPE: peptide
(xi) SEQUENCE DESCRIPTION: SEQ ID NO:7:
   Cys Asn Thr Arg Lys Ser Ile Lys Gly Pro Gly Arg Val Ile Tyr Ala
   1               5                   10                  15
   Thr Gly Gln

### (2) INFORMATION FOR SEQ ID NO:8:

(i) SEQUENCE CHARACTERISTICS:
   (A) LENGTH: 20 amino acids
   (B) TYPE: amino acid
   (D) TOPOLOGY: linear
(ii) MOLECULE TYPE: peptide
(xi) SEQUENCE DESCRIPTION: SEQ ID NO:8:
   Cys His Thr Arg Lys Arg Val Thr Leu Gly Pro Gly Arg Val Trp Tyr
   1               5                   10                  15
   Thr Thr Gly Glu
               20

### (2) INFORMATION FOR SEQ ID NO:9:

(i) SEQUENCE CHARACTERISTICS:
   (A) LENGTH: 20 amino acids
   (B) TYPE: amino acid
   (D) TOPOLOGY: linear
(ii) MOLECULE TYPE: peptide
(xi) SEQUENCE DESCRIPTION: SEQ ID NO:9:
   Cys Asn Thr Lys Lys Gly Ile Ala Ile Gly Pro Gly Arg Thr Leu Tyr
   1               5                   10                  15
   Ala Arg Glu Lys
               20

### (2) INFORMATION FOR SEQ ID NO:10:

(i) SEQUENCE CHARACTERISTICS:
   (A) LENGTH: 23 amino acids
   (B) TYPE: amino acid
   (D) TOPOLOGY: linear
(ii) MOLECULE TYPE: peptide
(xi) SEQUENCE DESCRIPTION: SEQ ID NO:10:
   Cys Asn Thr Arg Gln Ser Thr Pro Ile Gly Leu Gly Gln Ala Leu Tyr
   1               5                   10                  15
   Thr Thr Arg Gly Arg Thr Lys
               20

### (2) INFORMATION FOR SEQ ID NO:11:

(i) SEQUENCE CHARACTERISTICS:
   (A) LENGTH: 22 amino acids
   (B) TYPE: amino acid
   (D) TOPOLOGY: linear
(ii) MOLECULE TYPE: peptide
(xi) SEQUENCE DESCRIPTION: SEQ ID NO:11:
   Cys Asn Ile Arg Gln Arg Thr Ser Ile Gly Leu Gly Gln Ala Leu Tyr
   1               5                   10                  15
   Thr Thr Lys Thr Arg Ser
               20

### (2) INFORMATION FOR SEQ ID NO:12:

(i) SEQUENCE CHARACTERISTICS:
   (A) LENGTH: 22 amino acids
   (B) TYPE: amino acid
   (D) TOPOLOGY: linear
(ii) MOLECULE TYPE: peptide
(xi) SEQUENCE DESCRIPTION: SEQ ID NO:12:
   Cys Asn Thr Arg Gln Arg Thr Pro Ile Gly Leu Gly Gln Ser Leu Tyr
   1               5                   10                  15
   Thr Thr Arg Ser Arg Ser
               20

### (2) INFORMATION FOR SEQ ID NO:13:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(xi) SEQUENCE DESCRIPTION: SEQ ID NO:13:
   Arg Ile Thr Lys Gly Pro Gly Arg Val Ile Val Ala Thr Gly Gln
   1               5                   10                  15

### (2) INFORMATION FOR SEQ ID NO:14:

(i) SEQUENCE CHARACTERISTICS:
   (A) LENGTH: 402 base pairs
   (B) TYPE: nucleic acid
   (C) STRANDEDNESS: single
   (D) TOPOLOGY: linear
(ii) MOLECULE TYPE: DNA
(ix) FEATURE:
   (A) NAME/KEY: CDS
   (B) LOCATION: 1..402
(xi) SEQUENCE DESCRIPTION: SEQ ID NO:14:
   GAG GTC CAG CTG CAG GAG TCT GGA CCT GCT GTC ATC AAG CCA TCA CAG        48
   Glu Val Gln Leu Gln Glu Ser Gly Pro Ala Val Ile Lys Pro Ser Gln
     1               5                  10                  15
   TCA CTG TCT CTC ACC TGC ATA GTC TCT GGA TTC TCC ATC ACA AGT AGT        96
   Ser Leu Ser Leu Thr Cys Ile Val Ser Gly Phe Ser Ile Thr Ser Ser
                20                  25                  30
   AGT TAT TGC TGG CAC TGG ATC CGC CAG CCC CCA GGA AAG GGG TTA GAG       144
   Ser Tyr Cys Trp His Trp Ile Arg Gln Pro Pro Gly Lys Gly Leu Glu
            35                  40                  45
   TGG ATG GGG CGC ATA TGT TAT GAA GGT TCA ATA GAC TAT AGT CCA TCC       192
   Trp Met Gly Arg Ile Cys Tyr Glu Gly Ser Ile Asp Tyr Ser Pro Ser
        50                  55                  60
   ATC AAA AGC CGC AGC ACC ATC TCC AGA GAC ACA TCT CTG AAC AGA TTC       240
   Ile Lys Ser Arg Ser Thr Ile Ser Arg Asp Thr Ser Leu Asn Arg Phe
    65                  70                  75                  80
   TTT ATC CAG CTG AGT TCT GTG ACA AAT GAG GAC ACT GCC ATG TAT TAC       288
   Phe Ile Gln Leu Ser Ser Val Thr Asn Glu Asp Thr Ala Met Tyr Tyr
                    85                  90                  95
   TGT TCC AGG GAA AAC CAT GGT ACT ACG ACC TCT ATG GAC TAC TGG GGT       336
   Cys Ser Arg Glu Asn His Gly Thr Thr Thr Ser Met Asp Tyr Trp Gly
               100                 105                 110
   CAA GGA ACC TCA GTC ACC GTC TCC TCA GCC AAA ACA ACA CCC CCA TCA       384
   Gln Gly Thr Ser Val Thr Val Ser Ser Ala Lys Thr Thr Pro Pro Ser
           115                 120                 125
   GTC TAT CCA CTG GAA CCT
   Val Tyr Pro Leu Glu Pro
       130

### (2) INFORMATION FOR SEQ ID NO:15:

(i) SEQUENCE CHARACTERISTICS:
   (A) LENGTH: 134 amino acids
   (B) TYPE: amino acid
   (D) TOPOLOGY: linear
(ii) MOLECULE TYPE: protein
(xi) SEQUENCE DESCRIPTION: SEQ ID NO:15:
   Glu Val Gln Leu Gln Glu Ser Gly Pro Ala Val Ile Lys Pro Ser Gln
    1               5                  10                  15
   Ser Leu Ser Leu Thr Cys Ile Val Ser Gly Phe Ser Ile Thr Ser Ser
               20                  25                  30
   Ser Tyr Cys Trp His Trp Ile Arg Gln Pro Pro Gly Lys Gly Leu Glu
           35                  40                  45
   Trp Met Gly Arg Ile Cys Tyr Glu Gly Ser Ile Asp Tyr Ser Pro Ser
       50                  55                  60
   Ile Lys Ser Arg Ser Thr Ile Ser Arg Asp Thr Ser Leu Asn Arg Phe
   65                  70                  75                  80
   Phe Ile Gln Leu Ser Ser Val Thr Asn Glu Asp Thr Ala Met Tyr Tyr
                   85                  90                  95
   Cys Ser Arg Glu Asn His Gly Thr Thr Thr Ser Met Asp Tyr Trp Gly
               100                 105                 110
   Gln Gly Thr Ser Val Thr Val Ser Ser Ala Lys Thr Thr Pro Pro Ser
           115                 120                 125
   Val Tyr Pro Leu Glu Pro
       130

### (2) INFORMATION FOR SEQ ID NO:16:

(i) SEQUENCE CHARACTERISTICS:
   (A) LENGTH: 363 base pairs
   (B) TYPE: nucleic acid
   (C) STRANDEDNESS: single
   (D) TOPOLOGY: linear
(ii) MOLECULE TYPE: DNA
(ix) FEATURE:
   (A) NAME/KEY: CDS
   (B) LOCATION: 1..363
(xi) SEQUENCE DESCRIPTION: SEQ ID NO:16:
   GAC ATT GTG CTG ACC CAA TCT CCA GCT TCT TTG GCT GTG TCT CTA GGG      48
   Asp Ile Val Leu Thr Gln Ser Pro Ala Ser Leu Ala Val Ser Leu Gly
    1               5                  10                  15
   CAG AGG GCC ACC ATA TCC TGC AGA GCC AGT GAA AGT GTT GAT AGT TAT       96
   Gln Arg Ala Thr Ile Ser Cys Arg Ala Ser Glu Ser Val Asp Ser Tyr
               20                  25                  30
   GGC AAT AGT TTT ATG CAC TGG TAC CAG CAG AAA CCA GGA CAG TCA CCC      144
   Gly Asn Ser Phe Met His Trp Tyr Gln Gln Lys Pro Gly Gln Ser Pro
           35                  40                  45
   AAA CTC CTC ATC TAT GTT GCA TCC AAC CTA GAA TCT GGG GTC CCT GCC      192
   Lys Leu Leu Ile Tyr Val Ala Ser Asn Leu Glu Ser Gly Val Pro Ala
       50                  55                  60
   AGG TTC AGT GGC AGT GGG TCT AGG ACA GAC TTC ACC CTC ACC ATT GAT      240
   Arg Phe Ser Gly Ser Gly Ser Arg Thr Asp Phe Thr Leu Thr Ile Asp
   65                  70                  75                  80
   CCT GTG GAG GCT GAT GAT GCT GCA ACC TAT TAC TGT CAG CAA AAT AAT      288
   Pro Val Glu Ala Asp Asp Ala Ala Thr Tyr Tyr Cys Gln Gln Asn Asn
                   85                  90                  95
   GAG GAT CCG CTC GCG TTC GGT ACT GGG ACC AAG CTG GAG CTG AAA CGG      336
   Glu Asp Pro Leu Ala Phe Gly Thr Gly Thr Lys Leu Glu Leu Lys Arg
               100                 105                 110
   GCT GAT GCT GCA CCA ACT GTA TCC ATC                                  363
   Ala Asp Ala Ala Pro Thr Val Ser Ile
           115                 120

### (2) INFORMATION FOR SEQ ID NO:17:

(i) SEQUENCE CHARACTERISTICS:
   (A) LENGTH: 121 amino acids
   (B) TYPE: amino acid
   (D) TOPOLOGY: linear
(ii) MOLECULE TYPE: protein
(xi) SEQUENCE DESCRIPTION: SEQ ID NO:17:
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Glu Ser Val Asp Ser Tyr
               20                  25                  30
   Gly Asn Ser Phe Met His Trp Tyr Gln Gln Lys Pro Gly Gln Ser Pro
           35                  40                  45
   Lys Leu Leu Ile Tyr Val Ala Ser Asn Leu Glu Ser Gly Val Pro Ala
       50                  55                  60
   Arg Phe Ser Gly Ser Gly Ser Arg Thr Asp Phe Thr Leu Thr Ile Asp
   65                  70                  75                  80
   Pro Val Glu Ala Asp Asp Ala Ala Thr Tyr Tyr Cys Gln Gln Asn Asn
                   85                  90                  95
   Glu Asp Pro Leu Ala Phe Gly Thr Gly Thr Lys Leu Glu Leu Lys Arg
               100                 105                 110
   Ala Asp Ala Ala Pro Thr Val Ser Ile
           115                 120